# EUROPEAN PATENT SPECIFICATION

(11) **EP 2 988 671 B1**
(45) Date of publication and mention of the grant of the patent: **10.01.2024**
(21) Application number: 14721296.3
(22) Date of filing: 25.04.2014
(51) Int. Cl.: A61B 5/145, A61B 5/00, G01N 33/487

(54) **DIABETES MANAGEMENT SYSTEM WITH A MEDICAL DEVICE**
DIABETESMANAGEMENTSYSTEM MIT EINER MEDIZINISCHEN VORRICHTUNG
SYSTÈME DE PRISE EN CHARGE DU DIABÈTE AU MOYEN D'UN DISPOSITIF MÉDICAL

(30) Priority: 26.04.2013 US 201361816642 P; 26.04.2013 US 201361816659 P; 04.04.2014 US 201414245180
(43) Date of publication of application: 02.03.2016
(73) Proprietor: Roche Diabetes Care GmbH, 68305 Mannheim (DE); F. Hoffmann-La Roche AG, 4070 Basel (CH)
(72) Inventor: CARLSGAARD, Eric, Zionsville, IN 46077 (US); GEJDOS, Igor, Indianapolis, IN 46240 (US); KEPLEY, David M., Naperville, IL 60540 (US); LINDMAYER, Christian, 68782 Bruehl (DE); LONG, Michael Lee, Pendleton, IN 46064 (US); MC DANIEL, Angela S., Zionsville, IN 46077-7731 (US)
(74) Representative: Richardt Patentanwälte PartG mbB
(86) International application number: PCT/EP2014/058426
(87) International publication number: WO 2014/174063

(56) References cited:
- WO-A1-2012/108940
- WO-A2-2014/105631
- US-A1- 2006 031 094
- US-A1- 2010 331 627
- US-A1- 2011 178 820

## Description

### Field of the invention

The present disclosure relates to the use of device settings, medical and usage data, and statistics with a management system for patients with diabetes.

### Background and related art

This section provides background information related to the present disclosure which is not necessarily prior art.

Persons with diabetes often have difficulty regulating blood glucose levels in their bodies. As a consequence, many of these persons carry specialized portable medical devices, such as blood glucose meters, which allow them to periodically measure their glucose levels and take appropriate action, including administering insulin. After a blood glucose measurement or series of measurements is taken, a diabetic patient may find it useful to communicate these measurements to his or her health care professional for further review and analysis. In this regard, the patient's blood glucose meter may be capable of storing the blood glucose measurements for later review and analysis by the patient or the health care professional, who may then record the measurements manually or electronically.

The process of measuring, storing, recording, and analyzing blood glucose levels can be a very time consuming process for both the patient and the patient's health care professional. The exchange and review of data may require a meeting between the patient and the health care professional. People with diabetes are often searching for better and more efficient ways to manage their health. In addition, health care professionals need new ways to motivate people with diabetes to communicate more effectively. Technology can provide a viable platform for software applications for a wide variety of consumer demands. Moreover, many people with diabetes use personal computers and/or mobile devices in their daily lives.

WO 20121108940 describes a web-based software application that clinically analyzes multiple sources of diabetes patient data against codified care plan guidelines. The software application generates risk-based stratifications of a patient population, initiates rule-based notifications, allocates care provider resources, guides rolebased workflow, manages patient communications, and provides therapy recommendations.

US patent application US 20060031094A1 describes a system for managing data relating to one or more medical or biological conditions of a plurality of subjects (such as patients) over a wide area network, such as the Internet. The system may be employed for diabetes subjects or subjects with other medical conditions requiring monitoring and/or treatment over time. Such systems and processes provide various functions for several types of users, including patients or subject-users, healthcare provider-users and payor entity-users and combinations thereof, which allow for improved treatment and medical data management of individual subjects and groups of subjects and which allow collection and analysis of aggregate data from many subject sources, for improving overall healthcare practices.

US patent application US 20100331627A1 describes an adherence indication tool for chronic disease self-management and method thereof for measuring adherence or compliance to following or achieving prescribed therapy steps to achieve stated target goals for improved chronic disease self-management.

### Summary

This application claims the benefit of U.S. Provisional Application Nos. 61/816,642 and 61/816,659, both filed on April 26, 2013.

This section provides a general summary of the disclosure, and is not a comprehensive disclosure of its full scope or all of its features.

It is an object of the present invention to provide for an improved diabetes management system and corresponding method as specified in the independent claims. Various embodiments and enhancements thereto are given in the respective dependent claims. The embodiments and features described in the following paragraphs can freely be combined with each other if they are not mutually exclusive.

In one aspect the invention relates to a diabetes management system for monitoring multiple daily injection insulin treatments as specified in claim 1.

The system comprises a portable medical device associated with a patient and a data repository accessible by both the patient and a health care provider. The system further comprises a diabetes management application configured for receiving and storing, in the data repository, data from the portable medical device. The data may include medical data associated with the patient and usage data associated with usage of the portable medical device by the patient. The diabetes management application is further configured to accumulate and evaluate statistics related to the medical data or the usage data, and provide a treatment recommendation to the patient or the health care provider when the statistics meet or exceed a predetermined treatment criteria.

Said features may improve the effectiveness and efficiency of storing, communicating, and analyzing blood glucose measurements. The patient and the patient's health care professional may be enabled to send data, including blood glucose measurements, to a centralized electronic data repository for later retrieval and analysis. This may help to improve the management and treatment of diabetes.

Said features may also help to ensure compliance with a treatment regimen and improve glycemic control. Collecting and statistically evaluating usage data related to patient adherence may help to review and monitor treatment regimen adherence by the patient and/or the responsible health care provider.

In a further aspect, the present teachings provide a method for sharing information and managing diagnosis and treatment recommendations of a patient having a medical condition as specified in claim 12. The method comprises providing, at one or more servers, a web portal account accessible by both the patient and a health care provider. The web portal account may be associated with a portable medical device. The method includes receiving, at one or more servers, medical data and usage data from the portable medical device; the medical data may be associated with the patient and the usage data may be associated with usage of the medical device by the patient. Statistics related to the medical data or the usage data are accumulated at one or more servers. The statistics may be accumulated over a period of time and may be accessible in the web portal account. The method includes evaluating, at one or more servers, the statistics in relation to a treatment criteria; and sending, from one or more servers, a treatment recommendation to the patient or the health care provider when at least one of the statistics meets or exceeds a predetermined treatment criteria.

According to embodiments, the patient participates in a multiple daily injection insulin regimen as part of a diabetes treatment.

According to embodiments the statistics are indicative of at least one of a blood glucose measurement, a carbohydrate consumption, a bolus treatment, a basal rate, and adherence to a treatment recommendation.

According to embodiments the method further comprises accumulating statistics from a plurality of portable medical devices associated with the patient.

According to embodiments the method further comprises remotely adjusting, from one or more servers, at least one device setting on the portable medical device based on the treatment recommendation.

According to embodiments the device setting comprises at least one of an insulin:carbohydrate ratio, a basal rate, a maximum bolus amount, a calibration setting, a utility setting, and an alarm setting.

The usage data comprises data indicative of the patient's adherence to bolus treatment advice.

According to embodiments the treatment recommendation is sent as an electronic message to the portable medical device.

According to embodiments the method further comprises soliciting input from the health care provider prior to sending the treatment recommendation to the patient.

According to embodiments the method further comprises providing one or more proposed treatment recommendations to the health care provider, wherein the health care provider selects at least one of the proposed treatment recommendations for sending to the patient.

According to embodiments the treatment recommendation includes at least one of a reminder pertaining to eating habits, a reminder to transfer data from the portable medical device to the web portal account, a reminder to test blood glucose levels, a corrective basal rate change recommendation, and a corrective bolus dose recommendation.

According to embodiments data associated with the web portal account is accessible via the portable medical device.

In other aspects, the present teachings provide a method for diabetes management and treatment of a patient participating in a multiple daily injection insulin regimen. The method comprises providing, at one or more servers, a web portal account accessible by both the patient and a health care provider. The web portal account may be associated with a portable medical device. Medical data and usage data are received, at one or more servers, from the portable medical device. The medical data may be associated with the patient and the usage data may be associated with usage of the portable medical device by the patient. The method includes accumulating, at one or more servers, statistics related to the medical data or the usage data. The statistics may be accumulated over a period of time and are accessible in the web portal account. The statistics are evaluated, at one or more servers, in relation to a treatment criteria and may be associated with the web portal account for access by one or both of the patient and health care provider. The method includes providing a treatment recommendation to the patient or the health care provider when at least one statistic meets or exceeds a predetermined treatment criteria.

According to embodiments, the steps of providing the web portal account, receiving medical data and usage data, accumulating statistics related to the medical data or the usage data, evaluating the statistics and providing a treatment recommendation to the patient or the health care provider may be executed by the same set of one or more servers.

According to embodiments the treatment recommendation comprises an adjustment of a patient's insulin: carbohydrate ratio.

According to embodiments the method further comprises sending, from one or more servers, the treatment recommendation to the portable medical device as an electronic message.

According to embodiments the method further comprises remotely adjusting, from one or more servers, at least one device setting on the portable medical device based on the treatment recommendation.

According to embodiments the device setting comprises at least one of an insulin:carbohydrate ratio, a basal rate, a maximum bolus amount, a calibration setting, a utility setting, and an alarm setting.

According to embodiments the health care provider selects the statistics for evaluation.

According to embodiments, the method of any one of the above described embodiments is implemented and executed by one or more computers, e.g. one or more servers.

Further areas of applicability will become apparent from the description provided herein. The description and specific examples in this summary are intended for purposes of illustration only and are not intended to limit the scope of the present disclosure.

### Brief description of the drawings

The drawings described herein are for illustrative purposes only of selected embodiments and not all possible implementations, and are not intended to limit the scope of the present disclosure.
- Figure 1: is a diagram depicting an exemplary diabetes management system;
- Figure 2: is a diagram depicting a software architecture system for the diabetes management system;
- Figure 3: is a diagram depicting an alternative embodiment of the diabetes management system;
- Figure 4: is a diagram depicting an alternative embodiment of the software architecture system for the diabetes management system shown in Figure 3;
- Figure 5: is a diagram depicting an yet another alternative embodiment of the software architecture system for the diabetes management system shown in Figure 3;
- Figure 6: is a flowchart illustrating one embodiment of a method of sharing information and managing diagnosis of a patient with a medical condition;
- Figures 7-13: illustrate various graphical user interfaces that may be used in connection with the diabetes management system, management application, web browser, web portal account, and related software architecture; and
- Figure 14: is a diagram depicting the operation of an embodiment of a diabetes management system.

Corresponding reference numerals indicate corresponding parts throughout the several views of the drawings.

### Detailed description

Example embodiments will now be described more fully with reference to the accompanying drawings.

While the use of an insulin pump is manageable for many patients with diabetes, other patients decide to use a multiple daily injection regimen (MDI), which may allow more variation in lifestyle. With the variation in lifestyle, however, patients must be cognizant to monitor their diabetes regimen carefully, and be proactive with blood glucose testing and insulin administration. The present disclosure provides systems and methods for improved diabetes management, including the ability to detect, identify, and remedy certain situations where a patient with diabetes deviates from a prescribed therapy. In various aspects, when inconsistencies or discrepancies are detected, evaluated, or otherwise identified from the settings of a portable medical device and/or statistics related to or obtained from medical data and usage data, the systems and methods provided herein are able to assist in providing various treatment recommendations. The assistance may include evaluating medical data, usage data, and device settings, and determining one or more appropriate treatment recommendations for consideration by the patient or health care provider.

In various aspects, the diabetes management system can provide treatment recommendations that include setting changes for a portable medical device, changes in insulin administration and blood glucose testing, reminders, alerts, or even motivational statements based on adherence factors, with a goal to increase compliance with a treatment regimen and ultimately provide improved glycemic control.

Regimen adherence problems can be common with certain individuals with diabetes, often making glycemic control difficult to attain. Because the risk of complications of diabetes can be reduced by proper adherence, patient non-adherence to treatment recommendations can be frustrating for both diabetes patients and their health care professionals. Studies have shown that adherence rates for chronic illness regimens and for lifestyle changes are roughly about 50%. According to various implementations of the diabetes management system of the present disclosure, usage data related to patient adherence may be collected and evaluated by statistical analysis for review and monitoring by the patient and health care provider. Usage data related to adherence may include, for example, the number and timing of blood glucose measurements taken during a specified period; the number, amount, and timing of bolus treatments administered; the number, amount, and timing of corrective treatments administered; logging meal data and carbohydrate information, exercise routines, data uploads, illnesses, taking prescribed medications or medical treatments, attending medical appointments, behavioral changes, and the like.

For patients with an MDI basal-bolus diabetes treatment regime, basal and bolus treatments should be carefully configured and monitored. Basal rate determinations and corrections may vary based on both individual patient requirements and periods of change. For example, factors that may affect basal rates may include growth spurts, weight gain or loss, new or modified drug treatments, hormonal changes, eating changes and extended fasts, sleeping changes, exercise routine changes, long periods of inactivity, illness, stress, and seasonal changes. With respect to bolus insulin administration, carbohydrate counting and insulin-to-carbohydrate ratios are two important tools for matching insulin and food. To bolus properly, a diabetic patient will need to figure the approximate number of carbohydrates in each meal, and know his or her insulin-to-carbohydrate ratio. The portable medical device may be pre-programmed with the patient's specific ratio, which may be adjusted as needed. The insulin:carbohydrate ratio may be used to calculate the bolus dosage of insulin for each meal and/or snack.

By way of example, a person with diabetes with a regimen having an insulin:carbohydrate ratio of 1:5 would take 5 units of rapid-acting insulin as a bolus treatment to cover a meal containing 50 grams of carbohydrates, in addition to their basal insulin. This figure may often be determined on a trial-and-error basis, and for type 2 diabetics with severe insulin resistance, the numbers may be much higher. A standard ratio may be 1:15. By reviewing the statistics and historical data for a given patient, a health care provider may be able to provide updated treatment recommendations related to this ratio. In one example, a health care provider can look at an insulin:carbohydrate ratio and see that is has historically been set at 1:20. In another example, a patient may routinely have blood glucose levels within range before meals, but at about 2-3 hours post-meal, blood glucose levels remain high. This may be another example where it could be beneficial to decrease the insulin: carbohydrate ratio. If the patient's blood glucose levels are consistently not appropriate, the health care provider may be able to use the web portal of the present technology and look at statistics, usage data, device settings, and other factors such as exercise, food intake, and adherence data, and ultimately ascertain the patient is not getting enough insulin for the carbohydrates they are taking in. The health care provider can then provide a treatment recommendation adjusting the ratio to 1:15, for example, to compensate for the blood glucose levels. In certain aspects, the web portal is able to cooperate with the diabetes management system disclosed herein to provide collected statistics related to factors of interest to a health care provider, such as the patient exercise routines, food intake, and usage/adherence data. The various data may be uploaded to the web portal and diabetes management system by the patient.

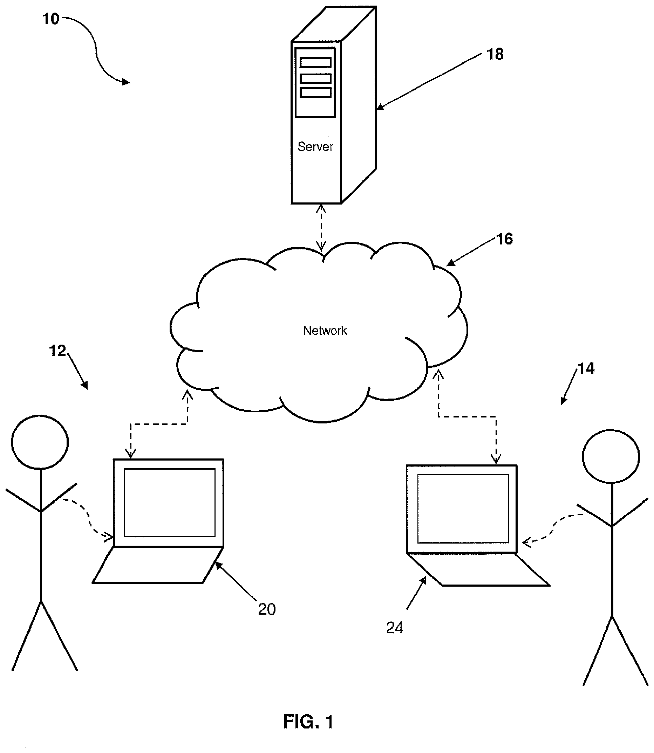

With reference to the above issues, and with the goal of advancing diabetes treatment, Figure 1 illustrates an exemplary diabetes management system ("DMS") 10 for storing and transmitting medical data, usage data, portable medical device settings, and accumulating and evaluating statistics and other relevant information available across a distributed computing environment. By way of non-limiting example, such medical data, usage data, and related information might include blood glucose measurements, carbohydrate consumption, basal rates, bolus treatments, adherence information and statistics, user and device settings, alerts and reminders, physician appointments, lab test results, user-entered notes, reports and graphs, and location information.

The diabetes management system 10 may generally include a patient data system 12, a health care professional data system 14, a network 16, and one or more server computers 18. As will be explained in more detail below, the diabetes management system 10 may be configured such that data and information is electronically sent and received to and from the patient data system 12, the health care professional data system 14, and the server computer 18 via the network 16.

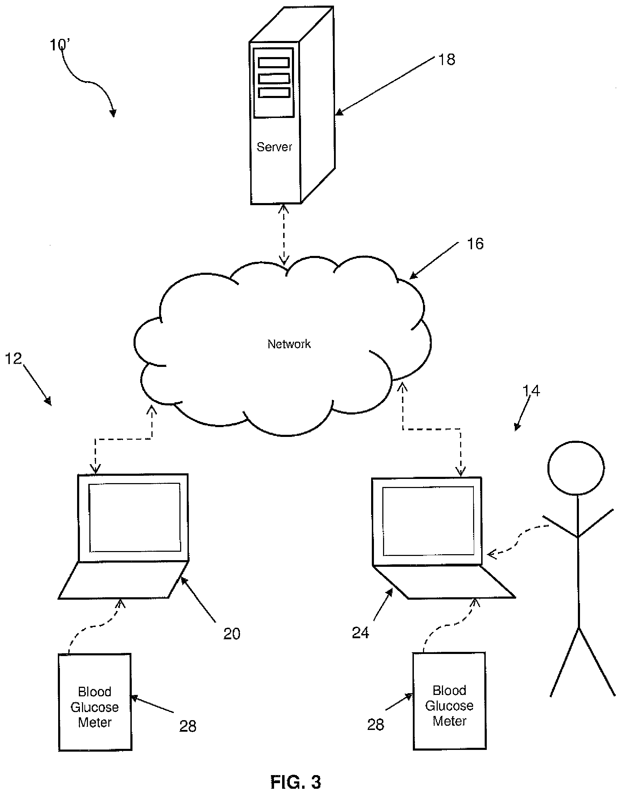

The diabetes management system 10' illustrated in Figure 3 may also generally include a portable medical device 28, such as a recording device or a blood glucose meter, for measuring and storing certain medical data or information, including blood glucose measurements.

The portable medical device 28 may be, for example, a handheld device that includes a port configured to receive a medical test strip having a reaction site for receiving a sample of fluid form a patient. The portable medical device 28 may include an internal blood glucose meter, cooperatively operable with a test strip inserted in the port and configured to measure glucose in a sample of fluid residing in the strip and associate identifying information with the glucose measurements. The portable medical device 28 may further include a user interface that selectively provides instructions, messages, and/or treatment recommendations to the patient. The portable medical device 28 may be paired with other portable devices, such as mobile communication devices, laptop computers, tablets, or other devices that are able to cooperate with the portable medical device 28 and display the instructions, messages, reminders, alerts, treatment recommendations, and the like.

The portable medical device 28 may be operable to send medical data, usage data, or other relevant information to at least one of the patient computing device 20 and the health care professional computing device 24 through a data transmission device, such as a hard-wired data port or a wireless data port such as a Bluetooth receiver, incorporated therein. The portable medical device 28 may be associated with a unique identifier or security code that attaches to any data transmitted by the portable medical device. Accordingly, in this method of data transmission, the patient or other user may not be required to enter a username, password, or other security code prior to transmitting data via the network 16.

It should be understood that patients may operate multiple portable devices in order to manage their diabetes treatments. The devices may be the same or different in function and/or design. It is envisioned that each device can be used with the diabetes management system, and multiple devices can transmit data manually or automatically.

The patient data system 12 may include at least one patient computing device 20 operably connected to, and in communication with, the network 16 through a wired or wireless connection such as WiFi. By way of example, the patient computing device 20 may be a desktop computer or a mobile communication device such as an electronic tablet or a smartphone. The patient computing device 20 may include a data input device, a processor, a memory, and an output device. The data input device may be a touchscreen, a keyboard, a mouse, a microphone, a hard-wired data port such as a universal serial bus port, or a wireless data port such as a Bluetooth receiver. The processor may be connected to the data input device, the memory, and the output device. In an example embodiment, the processor includes a general purpose processor. In another example embodiment, the processor includes an application specific integrated circuit. The output device includes a display, a speaker, or the like.

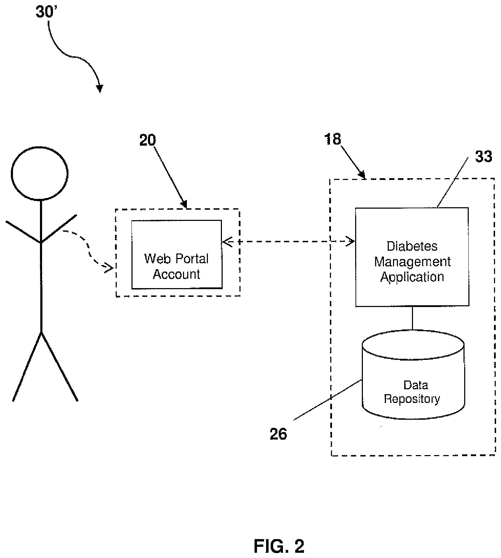

With reference to Figures 1 and 2, in one embodiment of the patient data system 12, a user (e.g., a patient) may enter medical data, usage data, or other relevant information, such as blood glucose measurements, into a web based system or account on the patient computing device 20 via the data input device for transmittal via the network 16. Prior to entering or transmitting data via the network 16, the user may be required to enter a username, password, or other security code, in order to ensure the accurate transmission and storage of data.

The health care professional data system 14 may include at least one health care professional computing device 24 operably connected to, and in communication with, the network 16 through a wired or wireless connection, such as WiFi. A physician or other person with appropriate credentials to access the health care professional computing device 24 may utilize the network 16 to download and view medical data, usage data, or other relevant information, such as blood glucose measurements, stored on the server computer 18. By way of example, the health care professional computing device 24 may be a desktop computer or a mobile communication device such as an electronic tablet or a smartphone.

The health care professional computing device 24 may include a data input device, a processor, a memory, and an output device. The data input device may be a touchscreen, a keyboard, a mouse, a microphone, a hard-wired data port such as a universal serial bus port, or a wireless data port such as a Bluetooth receiver. The processor may be connected to the data input device, the memory, and the output device. In an example embodiment, the processor includes a general purpose processor. In another example embodiment, the processor includes an application specific integrated circuit. The output device includes a display, a speaker, or the like.

With reference to Figures 1 and 2, one or more server computers 18 may include a processor, an input device, an output device, and a memory including a database or data repository 26. The processor is connected to the memory, the input device, and the output device. In an example embodiment, the processor includes a general purpose processor. In another example embodiment, the processor includes an application-specific integrated circuit. The input device includes a keyboard, a mouse, a touchpad, a trackpad, or the like. The output device includes a display, a speaker, or the like. The server computer 18 and database 26 may be operably connected to, and in communication with, the network 16 through a wired or wireless connection. The server computer 18 may be operable to send and receive via the network 16 data, such as blood glucose measurements received from the patient data system 12, to the database 26 for storage and later retrieval. As will be discussed below, data and information sent via the network 16 may be assigned to a unique web portal or equivalent patient account prior to being stored in the database 26 located on or otherwise accessible by the server computer 18.

Figure 3 illustrates an alternative embodiment of the diabetes management system 10'. The alternative embodiment of the diabetes management system 10' may be similar to the embodiment shown in Figure 1, and include a patient data system 12, a health care professional data system 14, a network 16, and at least one server computer 18. The patient data system 12 may include at least one patient computing device 20 operably connected to, and in communication with, the network 16 through a wired or wireless connection such as WiFi. The health care professional data system 14 may include at least one health care professional computing device 24 operably connected to, and in communication with, the network 16 through a wired or wireless connection, such as WiFi, through which a physician or other person with proper access to the health care professional computing device may download and view medical data, usage data, or other relevant information, such as blood glucose measurements, sent from the patient computing device 20 via the network 16.

The portable medical device 28 may be configured to transmit data to at least one of the patient computing device 20, the health care professional computing device 24, and one or more server computer 18 at regular, programmable intervals, at varying times chosen by the user, in real time as blood glucose measurements are taken, or when blood glucose measurements reach certain threshold device setting levels that may be set and remotely adjusted by the patient or the health care professional.

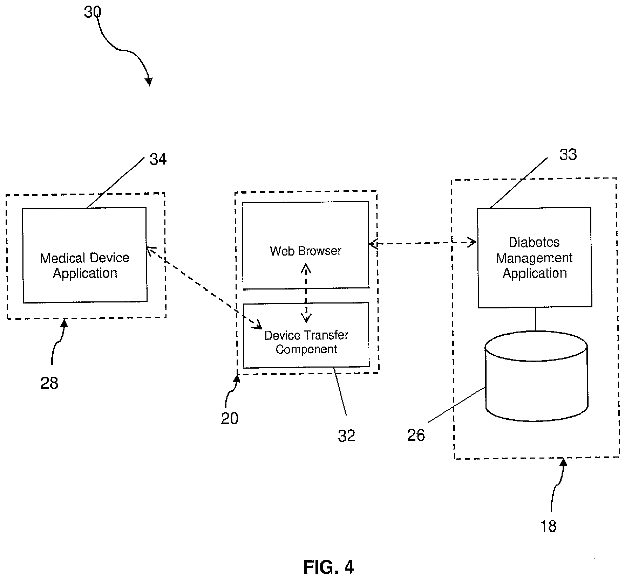

With reference to Figure 4, one embodiment of a software architecture system 30 for use with the diabetes management system 10 is illustrated. The software architecture system 30 may be operable to allow transmission and manipulation of medical data, usage data, and other relevant information, such as blood glucose measurements, from the portable medical device 28, through the network 16 and between the patient data system 12, the one or more server computer 18, and the health care professional data system 14.

The software architecture system 30 includes a device transfer component 32. The device transfer component 32 may be stored on the one or more server computer 18 for transmission through the network 16 and installation on the patient computing device 20 and the health care professional computing device 24. Installation of the device transfer component 32 onto the patient computing device 20 or the health care professional computing device 24 will allow such device to send data and information, such as blood glucose measurements, to the diabetes management application 33 installed on the server computer 18. When the portable medical device 28 sends data to the patient computing device 20 or the health care professional computing device 24, the device transfer component 32 is operable to pass the data to the server computer 18 via the network 16, without storing the data in the device transfer component 32 or the patient computing device 20. A medical device application 34 may be installed on the portable medical device 28, to facilitate the exchange of data and information with the device transfer component 32, as described above. Data sent via the device transfer component 32 may include an identifier code unique to the particular patient and/or portable medical device 28 from which it was received. The identifier code may allow the device transfer component 32 and the database 26 to efficiently send, store, and retrieve data from unique locations, or patient accounts, within the database 26.

Prior to transferring data and information through the network 16, a user may be required to link or associate the portable medical device 28 with a unique patient account created on or accessible by one or more server 18. Authentication may be accomplished by first allowing the portable medical device 28 to communicate with a patient computing device 20 via an input device such as a USB port or a Bluetooth receiver. The portable medical device 28 may be assigned a unique identification code or authentication token. After downloading and running the device transfer component 32, and allowing the portable medical device 28 to communicate with the personal computing device 20, the portable medical device's authentication token can be assigned to the appropriate patient account. Once the portable medical device's 28 authentication token has been assigned to the patient account, medical data, usage data, and other relevant information can be transferred through the device transfer component 32 and assigned to the appropriate patient account in the database 26.

In an alternative embodiment of the data transfer process, the device transfer component 32 may be assigned a unique identification code. Prior to transferring data and information from a portable medical device 28 through the network 16, the user may be required to associate the device transfer component's 32 unique identification code with the patient's account. Data and information from the portable medical device 28 can be linked to the device transfer component's 32 unique identification code prior to transmission through the network, and thus assigned to the appropriate patient account in the database 26.

After data has been transferred from the device transfer component 32 to the patient account located on the server 18, the software architecture system 30 may allow such data to be further transferred from the patient account to authorized health professional computing devices 24 and patient computing devices 20 via the device transfer component 32. Transfer of data from the patient account located in the database 26 on the server 18 may require linking the patient account with the device transfer component 32 installed on the health professional computing device 24.

The software architecture system 30 may also allow a patient, health care professional, or other user to create customized reports related to the data and information contained therein or perform research with respect to information contained within or outside of the software architecture system. In addition, the software architecture system 30 may be operable to provide data backup and restoration services with respect to data and information that may have been lost from the patient data system 12 or the health care professional data system 14.

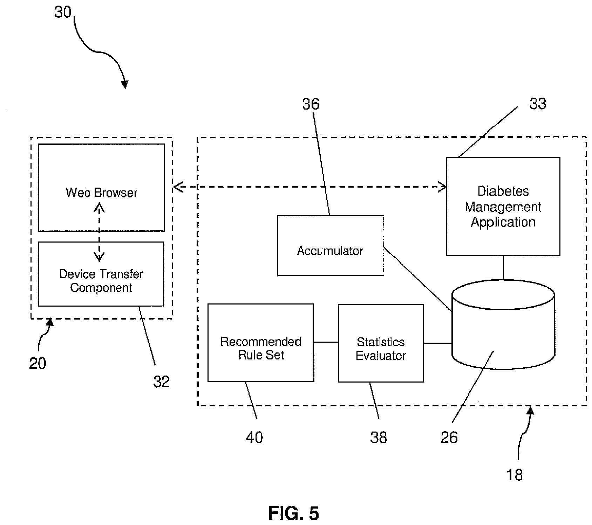

With reference to Figure 5, another embodiment of a software architecture system 30 for use with the diabetes management system 10 is illustrated. The database 26 on the server computer 18 may be in communication with an accumulator device or module 36 configured to accumulate statistics related to the medical data and usage data over a designated period of time. The database 26 may also be in communication with a statistics evaluator device or module 38, configured to collect, evaluate, and generate statistics related to the medical data and usage data for use with the diabetes management system 33. The statistics evaluator 38 may be in communication with a recommended rule set 40 that contains baseline information and standards related to the medical data and usage data being collected and analyzed, as well as information related to various treatment criteria that can be programmed therein. Treatment criteria may include minimum and maximum accepted values, as well as boundaries for normal and out-of-range data and/or statistics. In various aspects, the recommended rule set 40 may be updated or modified by an administrator or a health care provider. The accumulator device 36 and statistics evaluator 38 may be accessed through the diabetes management system application 33.

With reference to Figure 2, in another embodiment of the software architecture system 30', the user may send and receive data from one or more server computer 18 and the database 26 via a web browser on the patient computing device 20, in lieu of downloading and utilizing the device transfer component 32 on the patient computing device. In the software architecture system 30', the server computer 18 may include the device transfer component 32, allowing the user to send data and information to the server computer 18 for storage in the database 26, and for further transmission to the health care professional computing device 24 via the network 16. The user may access the diabetes management system 10 and the software architecture system 30' by entering a URL designated for the diabetes management system directly into a web browser.

Prior to transferring data through the network 16 to the server computer 18, the user may be required to create a unique patient account and security credentials, such as a username and password, to ensure a unique and secured storage location in the database 26. The user may also be required to associate, or link, the portable medical device 28 authentication token with the patient account. Once the user has created the patient account and linked the portable medical device 28 authentication code to the patient account, the user may be permitted to transfer data and information to and from the server computer 18 without otherwise logging into the patient account. In one aspect of the diabetes management system 10, access to the server 18 via the web browser may allow a user to transmit data such as blood glucose measurements, patient weight, meal information, and similar information, to the patient account in the database 26. In another aspect of the diabetes management system 10, access to the server 18 via the web browser may allow the user to create and view reports, graphs, and other information based at least in part on data transmitted from the user.

Communication between the patient data system 12, the health care professional data system 14, and the server computer 18 may utilize HTTP basic authentication in combination with secure sockets layer (SSL) security protocol. Other communication and security protocols known in the art are also contemplated.

In various aspects, the present technology uses a web portal in communication with the diabetes management system or management application to perform various functions related to health care provider accounts, patient accounts, or both. A web browser or web-based application may be used to perform similar functions. For example, a health care provider may be set up with a first, or main web portal account to help manage treatment of a plurality of different patients, each of which has a respective patient account accessible from the server computer associated with the diabetes management system. The health care provider may be able to configure its account to display certain data, statistics, and/or usage data from the various patients having web portal patient accounts accessible through the health care provider's account. A review of the data may allow the health care provider to better assess the patients' health, and adjust treatment regimens based on the recent and historical data.

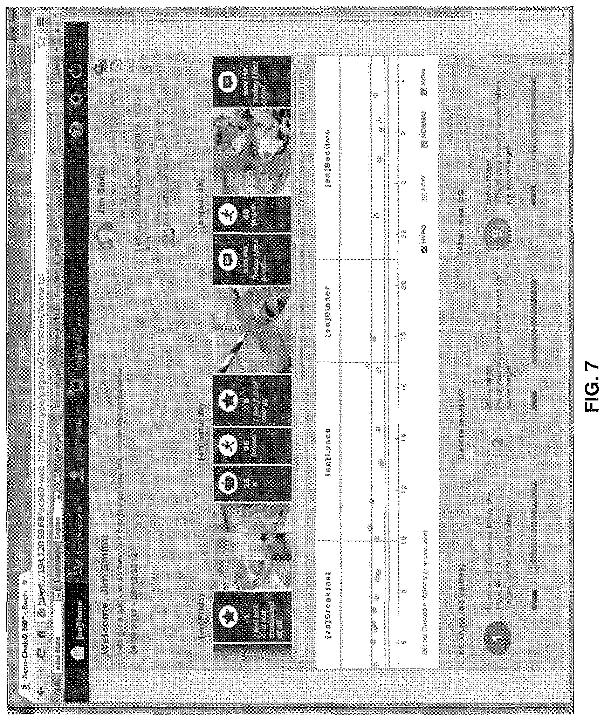

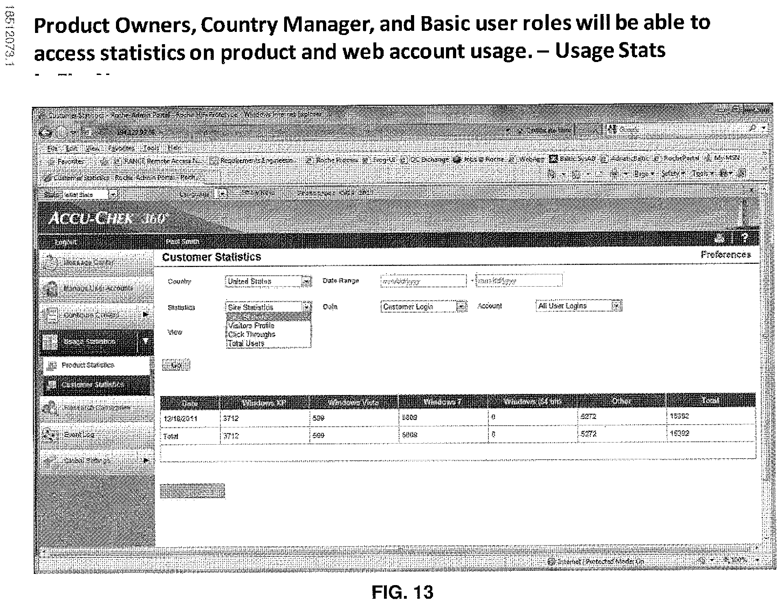

Figures 7-13 illustrate exemplary graphical user interfaces (GUI's) that may be used in connection with the diabetes management system, management application, web browser, web portal account, and related software architecture.

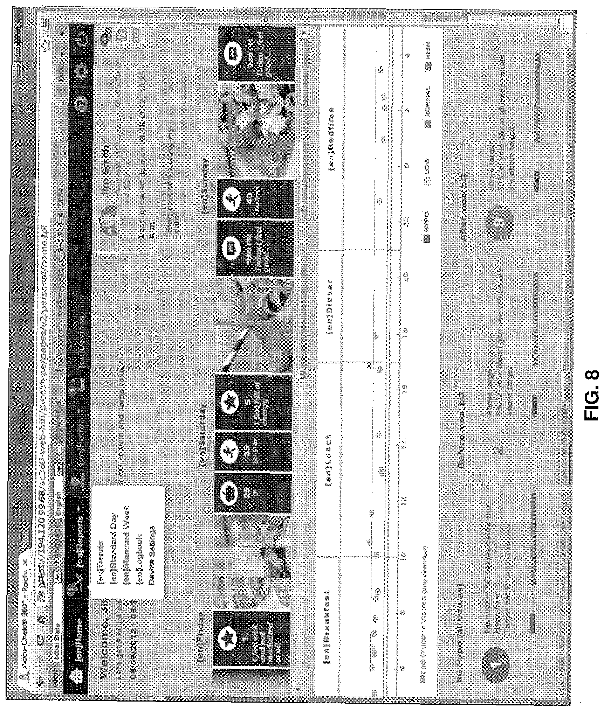

At various points during the use of the diabetes management system, access to information and the processing of requests or changes may be authenticated. Authentication may be accomplished using various techniques, including, but not limited to authentication of username and/or password credentials, permissions, unique identifiers, security questions, or other identifications. Authentication can also include solicitations or confirmations to one or more users seeking a confirmation or acknowledgement of the proposed request. Figures 7 and 8 illustrate a GUI welcome screen. In one example, the welcome screen may begin the authentication procedure, and subsequently provide access to account information, history, statistics, and an overview of selected features and data including blood glucose, insulin, and carbohydrate values manually entered, uploaded, or otherwise obtained from a handheld or portable medical device used by the patient.

In additional to typical logistical and testing information generally regarded as useful for diabetes treatment and management control, the web-portal may be configured to collect and use geographic locations of the health care provider and/or the patients in order to comply with certain regulatory and/or compliance regimens that may exist for different geographic locations, such as different states or countries. Various portions of the data such as references to medical patient data and personal data may be encrypted for patient security and confidentiality.

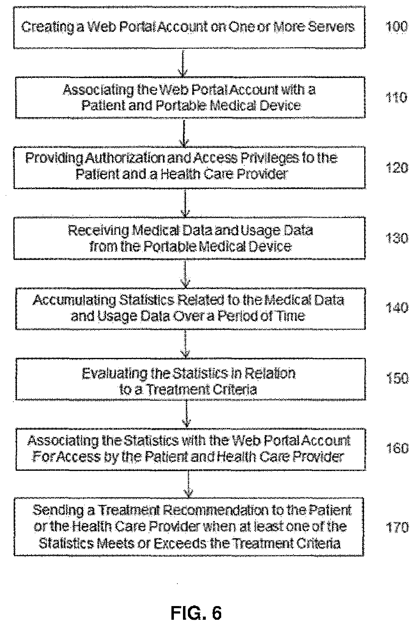

Figure 6 is a flowchart illustrating one embodiment of a method of sharing information and managing diagnosis of a patient with a medical condition. With reference to method step 100, a web portal account may be created on one or more servers. The account may be created by an appropriate administrator or health care provider. The web portal account may then be associated with a particular patient and/or a portable medical device, as shown in step 110. As discussed above, appropriate access privileges and authorization is provided to the patient and health care provider, as shown in step 120. In various aspects, a patient and/or health care provider with appropriate credentials (an authorized user) can access the various data stored within the diabetes management system. For example, an authorized user may review historical data and determine a treatment recommendation based on the data stored therein.

With reference to step 130, medical data and usage data is received from the portable medical device. In various aspects, the patient may log into the diabetes management system in order to manually enter or log medical data such as blood glucose measurements, insulin values, meal information, adherence evidence, or the like. The information may also be automatically transferred into the system by the patient or health care provider. As discussed above, a user such as the patient or health care provider may connect one or more portable medical devices to the diabetes management system and navigate to the device from within the system. The user may then select the appropriate device (if more than one device is connected) and instruct the system to transfer medical data, usage data, and/or user settings to the diabetes management system. Data may be transferred to the web portal account in a batch manner or after individual measurements are taken.

With reference to steps 140 and 150, statistics may be accumulated over period of time, where the accumulator device 36 and statistics evaluator 38 analyze medical data and usage data with respect to treatment criteria, as discussed above. The statistics may be associated with the appropriate web portal account, for access by the patient and the health care provider, as shown in step 160.

In various aspects, with reference to step 170, a treatment recommendation can be send to the patient or the health care provider when at least one of the statistics meets or exceeds the treatment criteria. The treatment recommendation may include instructing the patient to adjust user or device settings on one or more portable medical devices. The device settings may include, for example, when the device takes a measurement, when the device delivers insulin (if applicable), when the device reminds the patient to eat, the standard insulin:carbohydrate ratio, a basal rate, a maximum bolus amount, a calibration setting, a utility setting, and an alarm setting.

Various device settings may be uploaded to the diabetes management system. In certain aspects, at least one device setting of the portable medical devices may be remotely adjusted, for example from one or more servers, with the adjustment optionally being authorized, approved, or initiated by the health care provider based on a desired treatment recommendation. By way of example, the health care provider can adjust one or more settings using the web portal, and the next time the portable medical device is connected to the diabetes management system, the device setting will be synchronized with the portable medical device. In certain aspects, the logic in the diabetes management application may be programmed to solicit input from the health care provider prior to sending a treatment recommendation to the patient.

For example, one or more proposed treatment recommendations and/or suggested device setting changes may be made available to the health care provider, and the health care provider selects at least one proposed treatment recommendation for sending to the patient, or a device setting change to be made to the portable medical device. Once a device setting change is accepted and made to the device, the device may provide a confirmation message to the diabetes management system. A confirmation message may also be provided to the health care provider, for example, via a message, email, or alert made through the web portal, or directly to a health care provider through the use of a portable device.

In another example, a patient may upload multiple device settings to the diabetes management system. The upload process may be manual or automatic, depending on the portable medical device. The patient may grant access to the health care provider to view and adjust the device settings stored within the diabetes management system. The health care provider may connect to the diabetes management system via a web browser and a networked web portal account. After a review of the patient data, which may include a review of other relevant data in addition to device settings or usage data, such as food intake, exercise routines, etc., treatment recommendations can be submitted through the diabetes management system for display using the web portal account, and can also be provided directly to the portable medical device as an electronic message, alert, or reminder.

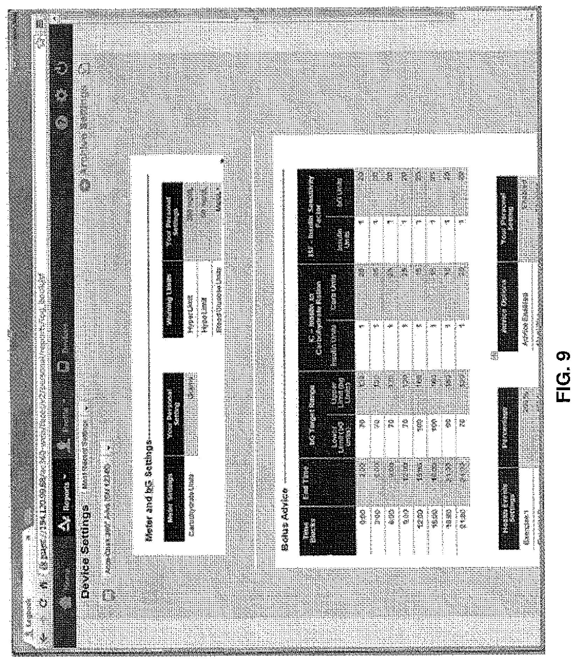

Figure 9 illustrates device settings for a particular portable medical device, such as the Accu-Check 360°. As shown, the device settings may include preferred measurement units and warning parameters, such as hyper and hypo blood glucose measurement limits. The device settings may include features related to bolus advice, including upper and lower limit blood glucose units, insulin:carbohydrate ratios, and insulin sensitivity factors. Various health event settings directed to exercise routines may be provided. Options may be available for a user to enable or disable certain features. Groups of settings may be saved as templates, and prior settings may be saved in an archive for later review, statistical analysis, and historical purposes.

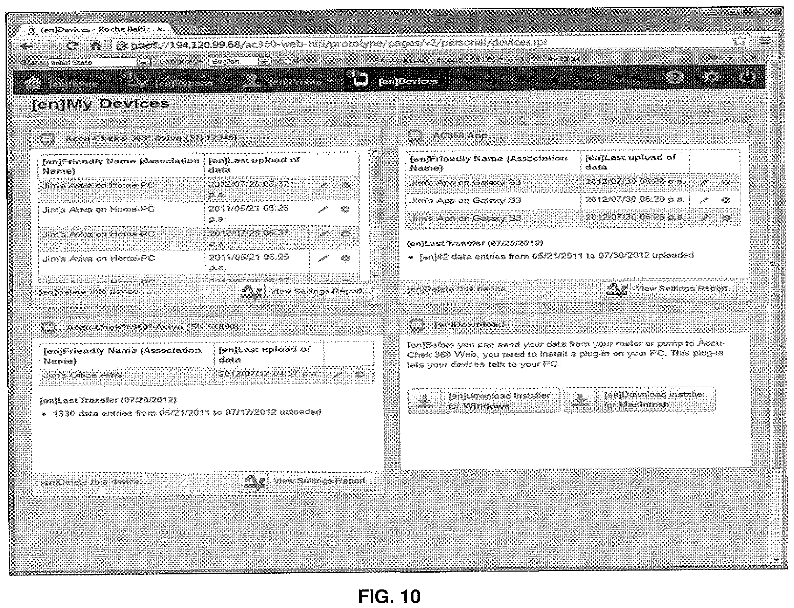

Figure 10 illustrates a summary interface for multiple devices used by a patient. In this regard, the patient may have a first portable medical device for home use and a second portable medical device for office use. As shown, a mobile communication device may also configured for this selected patient account. Each device may have its own settings and reports, and the interface may provide a listing of various data including last upload date and time for each device. In various aspects, the information displayed may be filtered, selected by a template, and otherwise customizable by the user. Software updates and installers may also be available through the interface.

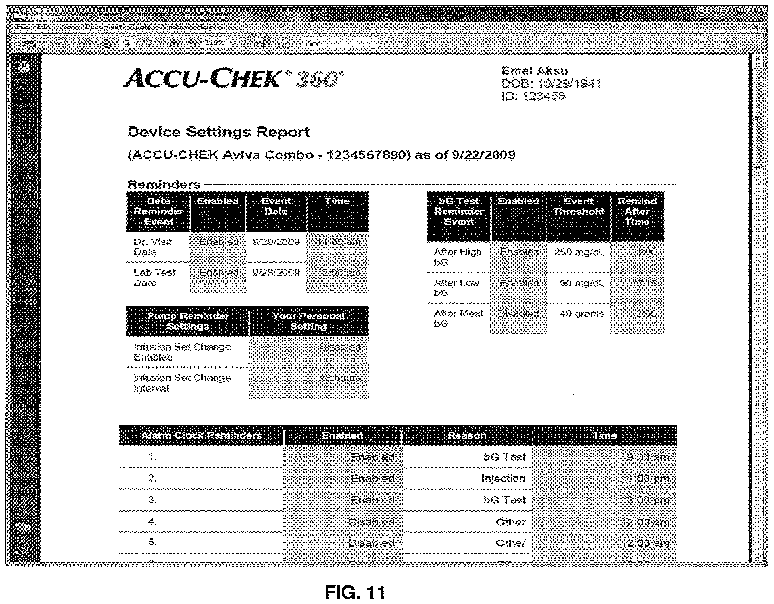

Figure 11 illustrates one example of a device settings report for a particular device. The report may include reminders of physician appointments, lab tests, pump reminder settings, testing reminder events, injection reminders, and other alarm features.

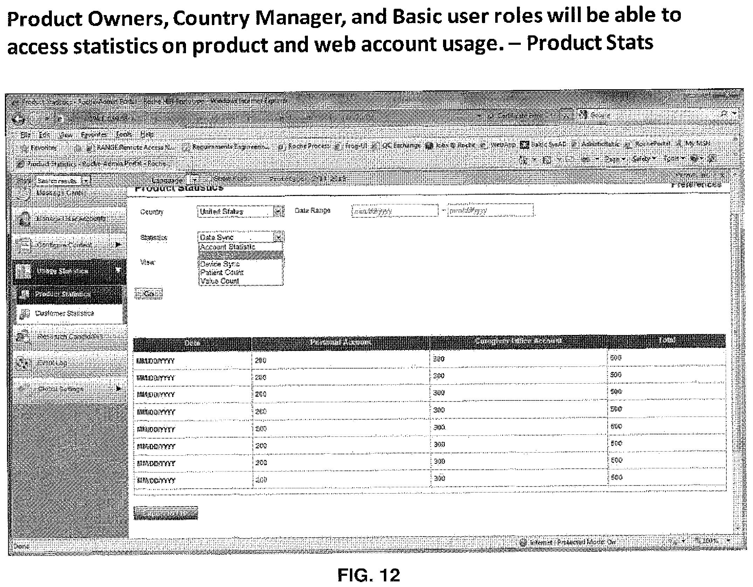

With reference to Figures 12-13, the diabetes management system may collect statistical data indicative of a number of times that medical data or usage data stored elsewhere in one or more portable medical devices was synchronized with data stored in the database or data repository. For example, one or more server computers can increment a first counter each time that medical data or usage data is uploaded or otherwise entered in the data repository. The value of the first counter may be displayed in response to a user request to view the number of times that data stored elsewhere was synchronized with data stored in the data repository. The counter can be filtered, for example, by date, device, type of data, etc.

In other aspects, the server computer may additionally or alternatively collect statistical data indicative of the number of times that the portable medical devices were synchronized with the diabetes management system. For example, a server computer may increment a second counter each time that a portable medical device is synchronized. The value of the second counter may be filtered and displayed in response to a patient or health care provider request to view the number of times the portable medical devices were synchronized.

The server computer may additionally or alternatively collect statistical data indicative of a number of patients that logged into their web portal accounts and/or reviewed messages or treatment recommendations provided to the patient via the web portal account. For example, a server computer may increment a third counter each time a patient logs into the web portal account. The value of the third counter may be filtered and displayed in response to a user request to view the number of times patients logged into their web portal account.

The server computer may additionally or alternatively collect statistical data indicative of the number of times the patient had interactions with the health care provider. For example, a server computer may increment a fourth counter each time that a patient had a visit or other interaction with the health care provider.

In various implementations, additional counters may be used, for example, with reminders and numerous adherence statistics, as discussed in more detail below. The server computer may reset the above-referenced counters in predetermined periods, such as once per day, week, etc. The predetermined periods can be updated as modified as necessary.

By utilizing the diabetes data management system, health care providers and patients may readily store and later access medical information relating to the patients, for example, to analyze historical information regarding a patient's biological condition, operation of the portable medical device, treatment, treatment results, personal habits, or the like. Based on such historical data, the health care provider and/or patient may be able to recognize trends, beneficial practices, detrimental practices, or the like and, thereby, adjust or design treatment plans that take advantage of beneficial trends and practices and avoids detrimental trends and practices. In certain aspects, the data management system includes certain preprogrammed logic that evaluates certain statistics and/or patient device settings and alerts patient or health care provider when the statistics or information is outside of a predetermined variance or treatment criteria.

Based on usage statistics received from one or more portable medical devices, the diabetes management system may collect and analyze information related to recommended amounts of insulin relative to amounts of insulin administered and/or a number of recommended blood glucose measurements taken per predetermined period (e.g., day, week) relative to an actual number of blood glucose measurements taken.

The diabetes management system may include software for generating or otherwise providing reports containing information received from a patient, a group of patients, or multiple groups of patients within the same health care provider account. In this manner, a patient or a patient's health care provider may readily access formatted reports of information regarding the patient's condition, historical condition, the patient portable medical device operation or condition, or the like, or similar information regarding one or more defined groups of patients. Reports may be formatted in various pre-defined formats provided by the diabetes management system. Alternatively or in addition, the system may allow patients and/or health care providers to design their own report format, including determining what type of information to include in the report and how the information is filtered, presented, displayed, etc. Various aspects of the present disclosure are directed a comprehensive system capable of collecting and managing patient information for multiple patients, the multiple patients with a plurality of different types of medical devices.

It should be understood that the sharing of accounts and data, as well as the maintaining of a suitable data repository can be managed by various administrative guidelines. Such administrative guidelines may need to follow certain procedures for opening, closing, and deactivating both patient and health care provider accounts associated with the data sharing and storage.

The web-portal or web-based browser/applications by which either the owners of the diabetes management system or the health care providers themselves perform various functions related to health care provider accounts, patient accounts, or both can be provided with certain predetermined procedures and/or parameters for use.

The techniques described herein may be implemented by one or more computer programs executed by one or more processors. The computer programs include processor-executable instructions that are stored on a non-transitory tangible computer readable medium. The computer programs may also include stored data. Non-limiting examples of the non-transitory tangible computer readable medium are nonvolatile memory, magnetic storage, and optical storage.

Some portions of the above description present the techniques described herein in terms of algorithms and symbolic representations of operations on information. These algorithmic descriptions and representations are the means used by those skilled in the data processing arts to most effectively convey the substance of their work to others skilled in the art. These operations, while described functionally or logically, are understood to be implemented by computer programs. Furthermore, it has also proven convenient at times to refer to these arrangements of operations as modules or by functional names, without loss of generality.

Unless specifically stated otherwise as apparent from the above discussion, it is appreciated that throughout the description, discussions utilizing terms such as "processing" or "computing" or "calculating" or "determining" or "displaying" or the like, refer to the action and processes of a computer system, or similar electronic computing device, that manipulates and transforms data represented as physical (electronic) quantities within the computer system memories or registers or other such information storage, transmission or display devices.

Certain aspects of the described techniques include process steps and instructions described herein in the form of an algorithm. It should be noted that the described process steps and instructions could be embodied in software, firmware or hardware, and when embodied in software, could be downloaded to reside on and be operated from different platforms used by real time network operating systems.

The present disclosure also relates to an apparatus for performing the operations herein. This apparatus may be specially constructed for the required purposes, or it may comprise a general-purpose computer selectively activated or reconfigured by a computer program stored on a computer readable medium that can be accessed by the computer. Such a computer program may be stored in a tangible computer readable storage medium, such as, but is not limited to, any type of disk including floppy disks, optical disks, CD-ROMs, magnetic-optical disks, read-only memories (ROMs), random access memories (RAMs), EPROMs, EEPROMs, magnetic or optical cards, application specific integrated circuits (ASICs), or any type of media suitable for storing electronic instructions, and each coupled to a computer system bus. Furthermore, the computers referred to in the specification may include a single processor or may be architectures employing multiple processor designs for increased computing capability.

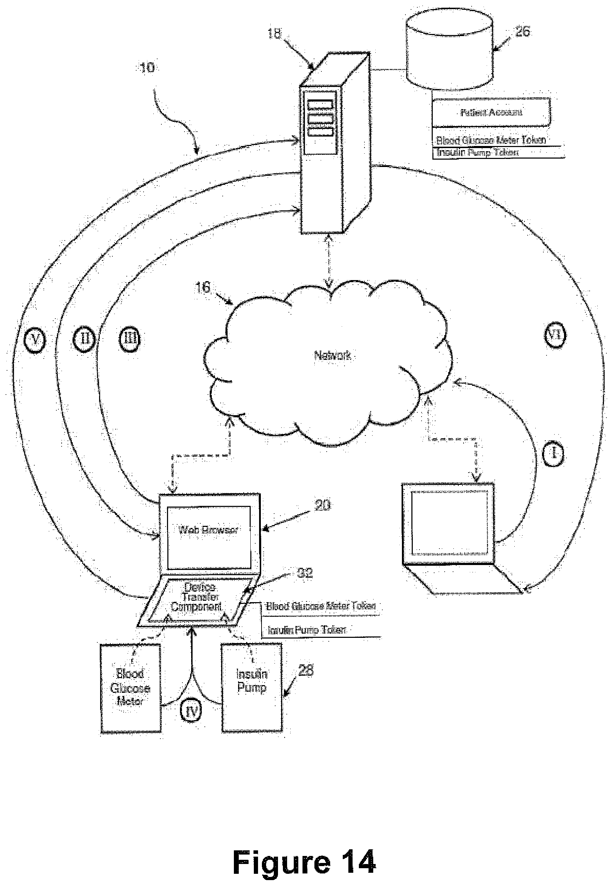

Figure 14 shows an embodiment of a diabetes management system. The server computer 18 may additionally or alternatively collect statistical data indicative of a number of user clicks (inputs). The number of user clicks may be collected, for example, based on the number of user clicks to a website serving the statistical data by the server computer 18 or a total number of user inputs to synchronized diabetes management devices. For example only, the server computer 18 may increment a fifth counter each time a user clicks a hyperlink of a website served by the server computer 18. The value of the fifth counter may be displayed in response to a user request to view the number user clicks to the website. In various implementations, the server computer 18 may reset the fifth counter each predetermined period, such as once per day.

The server computer 18 may additionally or alternatively collect statistical data indicative of how many times various computing device operating systems accessed the webpage served by the server computer 18. Example operating systems include, for example, Microsoft Windows XP, Microsoft Windows Vista, Microsoft Windows 7, Microsoft Windows 8, other versions of Microsoft Windows, Apple Mountain Lion, Apple iOS, Google Chrome, Google Android, Linux, and other types of operating systems. The server computer 18 may count the number of times the webpage was accessed by each different type of operating system. In various implementations, the server computer 18 may collect the cumulative number of times the webpage was accessed by two or more different operating systems. The numbers collected for one or more of operating systems may be displayed in response to a user request to view the number of accesses by portal type. In various implementations, the server computer 18 may reset the numbers each predetermined period, such as once per day.

### Examples

The present technology is further illustrated through the following non-limiting examples.

### Example 1

Upon review of the medical data and usage data collected for a particular patient in the web portal, a health care provider notices that the statistics for one patient indicate that he has had five hypo blood glucose measurements since the beginning of a new therapy adjustment from about one week ago. In certain aspects, the diabetes management application will have collected and analyzed the data, or statistics corresponding to the data, and may provide an alert to the health care provider that there have been multiple instances of hypo or hyper blood glucose readings. Alternatively, the diabetes management application may detect that there has been a discrepancy in usage data, for example, a user has not complied with the bolus advisor settings, or has not taken the appropriate amount of blood glucose measurements for a given time period.

In view of the above, the health care provider may send the patient an alert or a message. In one aspect, the alert may include a reminder to take blood glucose measurements. In another example, the alert may include instructions to perform a structured test. By way of example, the structured test may involve a "Testing in Pairs" ("TIP") program, where the patient is encouraged to focus on "before and after" testing, such as before and after meals, before and after exercise routines, before and after sleeping, etc. This type of testing program may help with a sense of cause-and-effect, and provides the ability to determine patterns in what may otherwise seemingly be "random" testing throughout the day. Appropriate logic for determining such patterns, as well as the metes and bounds for "normal" or "out-of-range" data may be programmed in the recommended rule set 40 as shown in Figure 5 and discussed above. Once the structured test is complete and the data is uploaded to the web portal and optionally analyzed and evaluated by the diabetes management system, the health care provider may be notified of the completed test.

In one example, the health care provider is able to review the relevant medical and usage data and statistics, ultimately ascertaining that the patient is having hypo measurements after breakfast. At the last appointment with the patient, the health care provider adjusted the insulin:carbohydrate ratio for breakfast in order to be more aggressive due to hyperglycemia after breakfast. The health care provider can now see the adjustment may have been too aggressive, and provides a less aggressive setting that is sent to the portable medical device, such as a bolus advisor, and sends an alert or message to the patient to perform further TIP for the breakfast meal for one week. Once the TIP is completed and the data is uploaded to the web portal, a notification may be provided to the health care provider. The health care provider may then review the resulting data and any statistics accumulated and/or evaluated by the diabetes management application in order to ascertain whether the issue has been resolved.

### Example 2

A health care provider filters all patient data associated with the health care provider account on the web portal in order to identify all patients with any lab test data that indicates an average level of blood sugar (glucose) over the previous three months (HbA1c) being greater than 7.5%. For many patients, a goal may be to keep this level at or below 6.5 - 7%. Of the patients having increased levels, the data in the web portal can be further filtered to determine which patients have not had an appointment with the health care provider within the last three months. This data can be obtained manually using the web portal, or the diabetes management system can be configured to provide the health care provider with a listing of those patients, and the health care provider can send each patient an alert or message to contact them to schedule a follow-up appointment. In certain aspects, the diabetes management system may be configured to automatically run such a query at predetermined times and/or intervals.

### Example 3

A health care provider has recently has made a therapy change for a patient and added a GLP-1 (glucagon-like peptide-1 agonist) to help control postprandial glucose values. The patient may be provided with instructions through the web portal or diabetes management application to perform pre- and postprandial tests for the most problematic meal of the day, breakfast. The health care provider may use the web portal to check the patient's medical and usage data for the next few days. In one example, the accumulated statistics indicate that the patient is still having high post breakfast glucose values. In certain aspects, this may be discovered by the diabetes management application comparing the usage and medical data to values in the recommended rule set and noting any out-of-range data. The health care provider may be informed of the situation by receiving an alert via the web portal, or the health care provider may review the data uploaded by the portable medical device. In response to the high glucose values, the health care provider may send an alert or message to the patient to increase their insulin dosage, and continue checking pre- and postprandial breakfast glucose. The health care provider may continue to check the portal after a few more days, and can see that the patient's post breakfast values are now within a normal range or target. The health care provider may send a follow-up alert, message, or note to the patient through the web portal to inform the patient that the revised dose is correct, and to follow up if the patient has any questions before the next office visit.

The foregoing description of embodiments and examples has been provided for purposes of illustration and description. It is not intended to be exhaustive or to limit the disclosure. Individual elements or features of a particular embodiment are generally not limited to that particular embodiment, but, where applicable, are interchangeable and can be used in a selected embodiment, even if not specifically shown or described. The same may also be varied in many ways. Such variations are not to be regarded as a departure from the disclosure, and all such modifications are intended to be included within the scope of the disclosure.

## Claims

1. A diabetes management system (10, 10', 30, 30') for monitoring multiple daily injection insulin treatments, comprising:
- a portable medical device (28) associated with a patient;
- a data repository (26) accessible by both the patient and a health care provider;
- a diabetes management application (33) configured for receiving and storing, in the data repository, data from the portable medical device, the data including medical data associated with the patient and usage data associated with usage of the portable medical device by the patient, wherein the usage data comprises data indicative of the patient's adherence to bolus treatment advice, said data comprising one or more of:
∘ the number and timing of blood glucose measurements taken during a specified period;
∘ the number, amount, and timing of bolus treatments administered;
∘ the number, amount, and timing of corrective treatments administered;
wherein the diabetes management system is configured for collecting statistical data indicative of a number of times that the medical data or usage data was synchronized with data stored in the data repository and for displaying said indicated number of times;
wherein the diabetes management application is configured to accumulate and evaluate statistics related to the usage data, and provide a treatment recommendation to the patient or the health care provider when the statistics meet or exceed a predetermined treatment criteria;
- a device transfer component (32), wherein the device transfer component is installed on a patient computing device (20) connected to one or more servers via a network (16), wherein the device transfer component enables the patient computing device to send the data from the portable medical device to the diabetes management application (33) installed on the one or more servers; wherein the data sent via the device transfer component includes an identifier code unique to the portable medical device (28) from which said data was received, whereby the device transfer component is configured to send said data to the one or more servers via the network and immediately erase any copy of the data from the device transfer component and/or from the patient computing device (20) after having successfully sent said data;
- the one or more servers (18), wherein the one or more servers are configured to execute steps comprising:
- receiving the data with the identifier code from the device transfer component;
- evaluating if said identifier code has already been stored in association with an account of said patient in the diabetes management application (33), wherein said storing indicates a successful registration of the portable medical device identified by said identifier code for said patient;
- if said identifier code has already been stored in association with said patient account, automatically storing the received data in the account of said patient in the data repository, thereby using the identification code as the solely necessary authentication data of the patient and automatically authenticating the patient at the diabetes management application via said identification code;
- if said identifier code has not already been stored in association with said patient account, prompting the patient via a user interface to enter authentication data; evaluating the entered authentication data; only in case of a successful authentication, automatically storing the received data in the account of said patient in the data repository, thereby storing the identification code in association with the account of the patient and automatically registering said portable medical device at the diabetes management application.

2. The diabetes management system according to any one of the previous claims, the one or more servers (18) being configured to share information and manage diagnosis and treatment of a patient having a medical condition, the one or more servers being configured to execute steps comprising:
- providing a web portal account accessible by both the patient and a health care provider, where the web portal account is associated with a portable medical device (28);
- receiving (130) medical data and usage data from the portable medical device, the medical data associated with the patient and the usage data indicative of usage of the portable medical device by the patient;
- accumulating (140) statistics related to the usage data, where the statistics are accumulated over a period of time and are accessible in the web portal account;
- evaluating (150) the statistics in relation to a treatment criteria; and
- sending (170) a treatment recommendation to the patient or the health care provider when at least one of the statistics meets or exceeds the treatment criteria.

3. The diabetes management system according to claim 2, wherein the statistics are indicative of at least one of a blood glucose measurement, a carbohydrate consumption, a bolus treatment, a basal rate, and adherence to a treatment recommendation.

4. The diabetes management system according to any one of the previous claims, wherein the one or more servers are further configured for accumulating statistics from a plurality of portable medical devices associated with the patient.

5. The diabetes management system according to any one of the previous claims 1-4, wherein the one or more servers are further configured for providing one or more proposed treatment recommendations to the health care provider, thereby enabling the health care provider to select at least one of the proposed treatment recommendations and send the selected treatment recommendations to a computing device (20) of the patient.

6. The diabetes management system according to any one of the previous claims, wherein the treatment recommendation includes at least one of a reminder pertaining to eating habits, a reminder to transfer data from the portable medical device to the web portal account, a reminder to test blood glucose levels, a corrective basal rate change recommendation, and a corrective bolus dose recommendation.

7. The diabetes management system according to any one of the previous claims, wherein data associated with the web portal account is accessible via the portable medical device.

8. The diabetes management system according to any one of the previous claims, the one or more servers (18) being configured to manage a multiple daily injection insulin regimen of a patient, the one or more servers being configured to execute steps comprising:
- providing a web portal account accessible by both the patient and a health care provider, where the web portal account is associated with a portable medical device (28);
- receiving (130) medical data and usage data from the portable medical device, the medical data associated with the patient and the usage data indicative of usage of the portable medical device by the patient;
- accumulating (140) statistics related to the usage data, where the statistics are accumulated over a period of time and are accessible in the web portal account;
- evaluating (150) the statistics in relation to a treatment criteria;
- associating (160) the statistics with the web portal account for access by one or both of the patient and health care provider; and
- providing a treatment recommendation to the patient or the health care provider when at least one of the statistics meets or exceeds a predetermined treatment criteria.

9. The diabetes management system according to any one of the previous claims 2-8, wherein the one or more servers are further configured to sending the treatment recommendation to the portable medical device as an electronic message.

10. The diabetes management system according to claim 9, wherein the one or more servers are further configured for soliciting input from the health care provider prior to sending the treatment recommendation to a computing device (20) of the patient.

11. The diabetes management system according to any one of the previous claims 2-10, wherein the one or more servers are configured such that the health care provider is enabled to select the type of statistics used for evaluation.

12. A method for sharing information and managing diagnosis-related data and treatment-recommendations of a patient having a medical condition, comprising:
- providing, at one or more servers (18), a web portal account accessible by both the patient and a health care provider, where the web portal account is associated with a portable medical device (28);
- receiving (130), at one or more servers, medical data and usage data from the portable medical device, the medical data associated with the patient and the usage data indicative of usage of the portable medical device by the patient, wherein the usage data is received via a device transfer component by the one or more servers and wherein the usage data includes an identifier code unique to the portable medical device (28) from which said usage data was received, wherein the usage data comprises data indicative of the patient's adherence to bolus treatment advice, said data comprising one or more of:
∘ the number and timing of blood glucose measurements taken during a specified period;
∘ the number, amount, and timing of bolus treatments administered;
∘ the number, amount, and timing of corrective treatments administered;
- immediately erasing, by the device transfer component, any copy of the data from the device transfer component after having successfully sent said data to the one or more servers;
- collecting statistical data indicating a number of times that medical data or usage data stored elsewhere in one or more portable medical devices was synchronized with data stored in the data repository for displaying said indicated number of times;
- evaluating, by the one or more servers, if said identifier code has already been stored in association with an account of said patient in the diabetes management application (33), wherein said storing indicates a successful registration of the portable medical device identified by said identifier code for said patient;
- if said identifier code has already been stored in association with said patient account, automatically storing, by the one or more servers, the received data in the account of said patient in the data repository, thereby using the identification code as the solely necessary authentication data of the patient and automatically authenticating the patient at the diabetes management application via said identification code;
- if said identifier code has not already been stored in association with said patient account, prompting, by the one or more servers, the patient via a user interface to enter authentication data; evaluating the entered authentication data; only in case of a successful authentication, automatically storing, by the one or more servers, the received data in the account of said patient in the data repository, thereby storing the identification code in association with the account of the patient and automatically registering said portable medical device at the diabetes management application;
- accumulating (140), at the one or more servers, statistics related to the usage data, where the statistics are accumulated over a period of time and are accessible in the web portal account;
- evaluating (150), at one or more servers, the statistics in relation to a treatment criteria; and
- sending (170), from one or more servers, a treatment recommendation to the patient or the health care provider when at least one of the statistics meets or exceeds the treatment criteria.

13. The method of claim 12 being implemented on a diabetes management system according to any one of claims 1-11.

## Patentansprüche

1. Diabetesmanagementsystem (10, 10', 30, 30') zum Überwachen mehrfacher täglicher Insulininjektionsbehandlungen, umfassend:
- eine tragbare medizinische Vorrichtung (28), die mit einem Patienten verbunden ist;
- einen Datenspeicher (26), auf den sowohl der Patient als auch ein Gesundheitsdienstleister Zugriff haben;
- eine Diabetesmanagementanwendung (33), die zum Empfangen und Speichern von Daten von der tragbaren medizinischen Vorrichtung in dem Datenspeicher konfiguriert ist, wobei die Daten medizinische Daten in Verbindung mit dem Patienten und Nutzungsdaten in Verbindung mit der Nutzung der tragbaren medizinischen Vorrichtung durch den Patienten einschließen, wobei die Nutzungsdaten Daten umfassen, die angeben, ob der Patient die empfohlene Bolusbehandlung befolgt, wobei die Daten eines oder mehreres von Folgendem umfassen:
o die Anzahl und Zeitpunkte von Blutzuckermessungen, die während eines vorgegebenen Zeitraums vorgenommen wurden,
o die Anzahl, Menge und Zeitpunkte von verabreichten Bolusbehandlungen;
o die Anzahl, Menge und Zeitpunkte von verabreichten Korrekturbehandlungen;
wobei das Diabetesmanagementsystem zum Erfassen statistischer Daten, die eine Anzahl von Fällen angeben, in denen die medizinischen Daten oder Nutzungsdaten mit Daten, die in dem Datenspeicher gespeichert sind, synchronisiert wurden, und zum Anzeigen der angegebenen Anzahl von Fällen konfiguriert ist;
wobei die Diabetesmanagementanwendung zum Sammeln und Auswerten von Statistiken bezüglich der Nutzungsdaten und Bereitstellen einer Behandlungsempfehlung dem Patienten oder dem Gesundheitsdienstleister konfiguriert ist, wenn die Statistiken vorbestimmte Behandlungskriterien erfüllen oder überschreiten;
- eine Vorrichtungstransferkomponente (32), wobei die Vorrichtungstransferkomponente an einer Patientenrechenvorrichtung (20) installiert ist, die über ein Netzwerk (16) mit einem oder mehreren Server(n) verbunden ist, wobei die Vorrichtungstransferkomponente der Patientenrechenvorrichtung ermöglicht, Daten von der tragbaren medizinischen Vorrichtung an die Diabetesmanagementanwendung (33), die auf dem einen oder den mehreren Server(n) installiert ist, zu senden; wobei die über die Vorrichtungstransferkomponente gesendeten Daten einen für die tragbare medizinische Vorrichtung (28), von der die Daten empfangen wurden, eindeutigen Identifizierungscode einschließen, wobei die Vorrichtungstransferkomponente zum Senden der Daten an den einen oder die mehreren Server über das Netzwerk und unmittelbaren Löschen von Kopien der Daten aus der Vorrichtungstransferkomponente und/oder aus der Patientenrechenvorrichtung (20), nachdem die Daten erfolgreich gesendet worden sind, konfiguriert ist;
- den einen oder die mehreren Server (18), wobei der eine oder die mehreren Server zur Ausführung von Schritten konfiguriert ist/sind, die Folgendes umfassen:
- Empfangen der Daten mit dem Identifizierungscode von der Vorrichtungstransferkomponente;
- Auswerten, ob der Identifizierungscode in Verbindung mit einem Konto des Patienten in der Diabetesmanagementanwendung (33) bereits gespeichert worden ist, wobei das Speichern eine erfolgreiche Registrierung der tragbaren medizinischen Vorrichtung, die von dem Identifizierungscode für den Patient identifiziert wurde, angibt;
- wenn der Identifizierungscode in Verbindung mit dem Patientenkonto bereits gespeichert worden ist, automatisches Speichern der empfangenen Daten in dem Konto des Patienten in dem Datenspeicher, wobei der Identifizierungscode als die einzigen notwendigen Authentifizierungsdaten des Patienten verwendet werden, und automatisches Authentifizieren des Patienten an der Diabetesmanagementanwendung mittels des Identifizierungscodes;
- wenn der Identifizierungscode nicht bereits in Verbindung mit dem Patientenkonto gespeichert worden ist, Auffordern des Patienten über eine Nutzerschnittstelle, Authentifizierungsdaten einzugeben; Auswerten der eingegebenen Authentifizierungsdaten; nur im Falle einer erfolgreichen Authentifizierung, automatisches Speichern der empfangenen Daten in dem Konto des Patienten in dem Datenspeicher, wobei der Identifizierungscode in Verbindung mit dem Konto des Patienten gespeichert und die tragbare medizinische Vorrichtung automatisch an der Diabetesmanagementanwendung registriert wird.

2. Diabetesmanagementsystem nach einem der vorstehenden Ansprüche, wobei der eine oder die mehreren Server (18) zum Teilen von Informationen und Managen der Diagnose und Behandlung eines Patienten mit einem medizinischen Leiden konfiguriert ist/sind, wobei der eine oder die mehreren Server zum Ausführen von Schritten konfiguriert ist/sind, die Folgendes umfassen:
- Bereitstellen eines Webportal-Kontos, auf das sowohl der Patient als auch ein Gesundheitsdienstleister Zugriff haben, wobei das Webportal-Konto mit einer tragbaren medizinischen Vorrichtung (28) verbunden ist;
- Empfangen (130) medizinischer Daten und Nutzungsdaten von der tragbaren medizinischen Vorrichtung, der medizinischen Daten in Verbindung mit dem Patienten und der Nutzungsdaten, die die Nutzung der tragbaren medizinischen Vorrichtung durch den Patienten angeben;
- Sammeln (140) von Statistiken bezüglich der Nutzungsdaten, wobei die Statistiken über einen Zeitraum gesammelt werden und in dem Webportal-Konto zugänglich sind;
- Auswerten (150) der Statistiken in Bezug auf Behandlungskriterien; und
- Senden (170) einer Behandlungsempfehlung an den Patienten oder den Gesundheitsdienstleister, wenn mindestens eine der Statistiken die Behandlungskriterien erfüllt oder überschreitet.

3. Diabetesmanagementsystem nach Anspruch 2, wobei die Statistiken mindestens eines von einer Blutzuckermessung, eines Kohlenhydratverbrauchs, einer Bolusbehandlung, einer Basalrate und Befolgung einer Behandlungsempfehlung angeben.

4. Diabetesmanagementsystem nach einem der vorstehenden Ansprüche, wobei der eine oder die mehreren Server ferner zum Sammeln von Statistiken von einer Vielzahl von tragbaren medizinischen Vorrichtungen in Verbindung mit dem Patienten konfiguriert ist/sind.

5. Diabetesmanagementsystem nach einem der vorstehenden Ansprüche 1-4, wobei der eine oder die mehreren Server ferner dazu konfiguriert ist/sind, dem Gesundheitsdienstleister eine oder mehrere vorgeschlagene Behandlungsempfehlungen bereitzustellen, wodurch dem Gesundheitsdienstleister ermöglicht wird, mindestens eine der vorgeschlagenen Behandlungsempfehlungen auszuwählen und die ausgewählten Behandlungsempfehlungen an eine Rechenvorrichtung (20) des Patienten zu senden.

6. Diabetesmanagementsystem nach einem der vorstehenden Ansprüche, wobei die Behandlungsempfehlung mindestens eine von einer die Essgewohnheiten betreffenden Erinnerung, einer Erinnerung, Daten von der tragbaren medizinischen Vorrichtung auf das Webportal-Konto zu übertragen, einer Erinnerung, Blutzuckerspiegel zu testen, einer Korrekturempfehlung zur Änderung der Basalrate und einer Korrekturempfehlung für die Bolusdosis einschließt.

7. Diabetesmanagementsystem nach einem der vorstehenden Ansprüche, wobei Daten in Verbindung mit dem Webportal-Konto über die tragbare medizinische Vorrichtung zugänglich sind.

8. Diabetesmanagementsystem nach einem der vorstehenden Ansprüche, wobei der eine oder die mehreren Server (18) zum Managen eines Regimes von mehrfachen täglichen Insulininjektionen eines Patienten konfiguriert ist/sind, wobei der eine oder die mehreren Server zum Ausführen von Schritten konfiguriert ist/sind, die Folgendes umfassen:
- Bereitstellen eines Webportal-Kontos, auf das sowohl der Patient als auch ein Gesundheitsdienstleister Zugriff haben, wobei das Webportal-Konto mit einer tragbaren medizinischen Vorrichtung (28) verbunden ist;
- Empfangen (130) medizinischer Daten und Nutzungsdaten von der tragbaren medizinischen Vorrichtung, der medizinischen Daten in Verbindung mit dem Patienten und der Nutzungsdaten, die die Nutzung der tragbaren medizinischen Vorrichtung durch den Patienten angeben;
- Sammeln (140) von Statistiken bezüglich der Nutzungsdaten, wobei die Statistiken über einen Zeitraum gesammelt werden und in dem Webportal-Konto zugänglich sind;
- Auswerten (150) der Statistiken in Bezug auf Behandlungskriterien;
- Inverbindungbringen (160) der Statistiken mit dem Webportal-Konto für Zugriff durch einen oder beide von dem Patienten und dem Gesundheitsdienstleister; und
- Bereitstellen einer Behandlungsempfehlung dem Patienten oder dem Gesundheitsdienstleister, wenn mindestens eine der Statistiken vorbestimmte Behandlungskriterien erfüllt oder überschreitet.

9. Diabetesmanagementsystem nach einem der vorstehenden Ansprüche 2-8, wobei der eine oder die mehreren Server ferner zum Senden der Behandlungsempfehlung an die tragbare medizinische Vorrichtung als eine elektronische Nachricht konfiguriert ist/sind.

10. Diabetesmanagementsystem nach Anspruch 9, wobei der eine oder die mehreren Server ferner zum Abrufen einer Eingabe des Gesundheitsdienstleisters vor dem Senden der Behandlungsempfehlung an eine Rechenvorrichtung (20) des Patienten konfiguriert ist/sind.

11. Diabetesmanagementsystem nach einem der vorstehenden Ansprüche 2-10, wobei der eine oder die mehreren Server so konfiguriert ist/sind, dass dem Gesundheitsdienstleister ermöglicht wird, die Art von Statistiken, die zur Auswertung verwendet werden, auszuwählen.

12. Verfahren zum Teilen von Informationen und Managen diagnosebezogener Daten und Behandlungsempfehlungen eines Patienten mit einem medizinischen Leiden, umfassend:
- Bereitstellen eines Webportal-Kontos, auf das sowohl der Patient als auch ein Gesundheitsdienstleister Zugriff haben, auf einem oder mehreren Server(n) (18), wobei das Webportal-Konto mit einer tragbaren medizinischen Vorrichtung (28) verbunden ist;
- Empfangen (130) medizinischer Daten und Nutzungsdaten von der tragbaren medizinischen Vorrichtung, der medizinischen Daten in Verbindung mit dem Patienten und der Nutzungsdaten, die die Nutzung der tragbaren medizinischen Vorrichtung durch den Patienten angeben, auf einem oder mehreren Server(n), wobei die Nutzungsdaten von dem einen oder den mehreren Server(n) über eine Vorrichtungstransferkomponente empfangen werden und wobei die Nutzungsdaten einen für die tragbare medizinische Vorrichtung (28), von der die Nutzungsdaten empfangen wurden, eindeutigen Identifizierungscode einschließen, wobei die Nutzungsdaten Daten umfassen, die angeben, ob der Patient die empfohlene Bolusbehandlung befolgt, wobei die Daten eines oder mehreres von Folgendem umfassen:
∘ die Anzahl und Zeitpunkte von Blutzuckermessungen, die während eines speziellen Zeitraumes vorgenommen wurden;
∘ die Anzahl, Menge und Zeitpunkte von verabreichten Bolusbehandlungen;
∘ die Anzahl, Menge und Zeitpunkte von verabreichten Korrekturbehandlungen;
- unmittelbares Löschen von Kopien der Daten von der Vorrichtungstransferkomponente mittels der Vorrichtungstransferkomponente, nachdem die Daten erfolgreich an den einen oder die mehreren Server gesendet worden sind;
- Erfassen statistischer Daten, die eine Anzahl von Fällen angeben, in denen medizinische Daten oder Nutzungsdaten, die woanders in einer oder mehreren tragbaren medizinischen Vorrichtungen gespeichert sind, mit Daten, die in dem Datenspeicher gespeichert sind, synchronisiert wurden, zum Anzeigen der angegebenen Anzahl von Fällen;
- Auswerten, ob der Identifizierungscode in Verbindung mit einem Konto des Patienten bereits in der Diabetesmanagementanwendung (33) gespeichert worden ist, mittels des einen oder der mehreren Server(s), wobei das Speichern eine erfolgreiche Registrierung der tragbaren medizinischen Vorrichtung, die von dem Identifizierungscode für den Patienten identifiziert wurde, angibt;
- wenn der Identifizierungscode in Verbindung mit dem Patientenkonto bereits gespeichert worden ist, automatisches Speichern der empfangenen Daten in dem Konto des Patienten in dem Datenspeicher mittels des einen oder der mehreren Server(s), wobei der Identifizierungscode als die einzigen notwendigen Authentifizierungsdaten des Patienten verwendet werden, und automatisches Authentifizieren des Patienten an der Diabetesmanagementanwendung mittels des Identifizierungscodes;
- wenn der Identifizierungscode nicht bereits in Verbindung mit dem Patientenkonto gespeichert worden ist, Auffordern des Patienten über eine Nutzerschnittstelle mittels des einen oder der mehreren Server(s), Authentifizierungsdaten einzugeben; Auswerten der eingegebenen Authentifizierungsdaten; nur im Falle einer erfolgreichen Authentifizierung, automatisches Speichern der empfangenen Daten in dem Konto des Patienten in dem Datenspeicher mittels des einen oder der mehreren Server(s), wobei der Identifizierungscode in Verbindung mit dem Konto des Patienten gespeichert und die tragbare medizinische Vorrichtung automatisch an der Diabetesmanagementanwendung registriert wird;
- Sammeln (140) von Statistiken bezüglich der Nutzungsdaten auf dem einen oder den mehreren Server(n), wobei die Statistiken über einen Zeitraum gesammelt werden und in dem Webportal-Konto zugänglich sind;
- Auswerten (150) der Statistiken in Bezug auf Behandlungskriterien auf einem oder mehreren Server(n); und
- Senden (170) einer Behandlungsempfehlung von einem oder mehreren Server(n) an den Patienten oder den Gesundheitsdienstleister, wenn mindestens eine der Statistiken die Behandlungskriterien erfüllt oder überschreitet.

13. Verfahren Anspruch 12, das auf einem Diabetesmanagementsystem nach einem der Ansprüche 1-11 implementiert ist.

## Revendications

1. Système de prise en charge du diabète (10, 10', 30, 30') pour la surveillance des traitements d'insuline par injections quotidiennes multiples, comprenant :
- un dispositif médical portable (28) associé à un patient ;
- un référentiel de données (26) accessible à la fois par le patient et un professionnel de santé ;
- une application de prise en charge du diabète (33) configurée pour recevoir et stocker, dans le référentiel de données, des données provenant du dispositif médical portable, les données incluant des données médicales associées au patient et des données d'usage associées à l'usage du dispositif médical portable par le patient, dans lequel les données d'usage comprennent des données indiquant l'adhésion du patient à un conseil de traitement par bolus, lesdites données comprenant un ou plusieurs parmi :
∘ le nombre et la chronologie des mesures de glycémie prises pendant une période spécifiée ;
∘ le nombre, la quantité et la chronologie des traitements par bolus administrés ;
∘ le nombre, la quantité et la chronologie des traitements correctifs administrés ;
dans lequel le système de prise en charge du diabète est configuré pour recueillir des données statistiques indiquant un nombre de fois que les données médicales ou les données d'usage ont été synchronisées avec les données stockées dans le référentiel de données et pour l'affichage dudit nombre de fois indiqué ;
dans lequel l'application de prise en charge du diabète est configurée pour accumuler et évaluer des statistiques liées aux données d'usage, et fournir une recommandation de traitement au patient ou au professionnel de santé lorsque les statistiques correspondent à ou dépassent un critère de traitement prédéterminé ;
- un composant de transfert de dispositif (32), dans lequel le composant de transfert de dispositif est installé sur un dispositif informatique du patient (20) connecté à un ou plusieurs serveurs par l'intermédiaire d'un réseau (16), dans lequel le composant de transfert de dispositif permet au dispositif informatique du patient d'envoyer les données du dispositif médical portable jusqu'à l'application de prise en charge du diabète (33) installée sur le ou les serveurs ; dans lequel les données envoyées par l'intermédiaire du composant de transfert de dispositif incluent un code identifiant unique au dispositif médical portable (28) duquel lesdites données ont été reçues, moyennant quoi le composant de transfert de dispositif est configuré pour envoyer lesdites données au ou aux serveurs par l'intermédiaire du réseau et effacer immédiatement une quelconque copie des données provenant du composant de transfert de dispositif et/ou du dispositif informatique du patient (20) après avoir envoyé lesdites données avec succès ;
- le ou les serveurs (18), dans lequel le ou les serveurs sont configurés pour exécuter les étapes comprenant :
- la réception des données avec le code identifiant provenant du composant de transfert de dispositif ;
- l'évaluation si ledit code identifiant a déjà été stocké en association avec un compte dudit patient dans l'application de prise en charge du diabète (33), dans lequel ledit stockage indique un enregistrement réussi du dispositif médical portable identifié par ledit code identifiant pour ledit patient ;
- si ledit code identifiant a déjà été stocké en association avec ledit compte patient, le stockage de manière automatique des données reçues dans le compte dudit patient dans le référentiel de données, utilisant de ce fait le code d'identification en tant que seule donnée d'identification nécessaire du patient et authentifiant de manière automatique le patient au niveau de l'application de prise en charge du diabète par l'intermédiaire dudit code d'identification ;
- si ledit code identifiant n'a pas déjà été stocké en association avec ledit compte patient, l'invite faite au patient par l'intermédiaire d'une interface utilisateur de saisir les données d'authentification ; l'évaluation des données d'authentification saisies ; uniquement dans le cas d'une authentification réussie, le stockage de manière automatique des données reçues dans le compte dudit patient dans le référentiel de données, stockant de ce fait le code d'identification en association avec le compte du patient et enregistrant de manière automatique ledit dispositif médical portable au niveau de l'application de prise en charge du diabète.

2. Système de prise en charge du diabète selon l'une quelconque des revendications précédentes, le ou les serveurs (18) étant configurés pour partager des informations et prendre en charge le diagnostic et le traitement d'un patient souffrant d'une affection médicale, le ou les serveurs étant configurés pour exécuter les étapes comprenant :
- la fourniture d'un compte de portail web accessible à la fois par le patient et un professionnel de santé, où le compte de portail web est associé à un dispositif médical portable (28) ;
- la réception (130) de données médicales et de données d'usage provenant du dispositif médical portable, les données médicales étant associées au patient et les données d'usage indiquant l'usage du dispositif médical portable par le patient ;
- l'accumulation (140) de statistiques liées aux données d'usage, où les statistiques sont accumulées sur une période de temps et sont accessibles dans le compte de portail web ;
- l'évaluation (150) des statistiques en lien avec un critère de traitement ; et
- l'envoi (170) d'une recommandation de traitement au patient ou au professionnel de santé lorsqu'au moins l'une des statistiques correspond au ou dépasse le critère de traitement.

3. Système de prise en charge du diabète selon la revendication 2, dans lequel les statistiques indiquent au moins un parmi une mesure de glycémie, une consommation de glucides, un traitement par bolus, un débit basal et une adhésion à une recommandation de traitement.

4. Système de prise en charge du diabète selon l'une quelconque des revendications précédentes, dans lequel le ou les serveurs sont en outre configurés pour accumuler des statistiques provenant d'une pluralité de dispositifs médicaux portables associés au patient.

5. Système de prise en charge du diabète selon l'une quelconque des revendications 1 à 4 précédentes, dans lequel le ou les serveurs sont en outre configurés pour fournir une ou plusieurs recommandations de traitement proposées au professionnel de santé, permettant de ce fait au professionnel de santé de sélectionner au moins une des recommandations de traitement proposées et d'envoyer les recommandations de traitement sélectionnées à un dispositif informatique (20) du patient.

6. Système de prise en charge du diabète selon l'une quelconque des revendications précédentes, dans lequel la recommandation de traitement inclut au moins un parmi un rappel concernant les habitudes alimentaires, un rappel pour transférer des données du dispositif médical portable jusqu'au compte de portail web, un rappel pour tester les taux de glycémie, une recommandation de changement de débit basal correctif et une recommandation de dose de bolus corrective.

7. Système de prise en charge du diabète selon l'une quelconque des revendications précédentes, dans lequel les données associées au compte de portail web sont accessibles par l'intermédiaire du dispositif médical portable.

8. Système de prise en charge de diabète selon l'une quelconque des revendications précédentes, le ou les serveurs (18) étant configurés pour prendre en charge une posologie d'insuline par injections quotidiennes multiples d'un patient, le ou les serveurs étant configurés pour exécuter les étapes comprenant :
- la fourniture d'un compte de portail web accessible à la fois par le patient et un professionnel de santé, où le compte de portail web est associé à un dispositif médical portable (28) ;
- la réception (130) de données médicales et de données d'usage provenant du dispositif médical portable, les données médicales étant associées au patient et les données d'usage indiquant l'usage du dispositif médical portable par le patient ;
- l'accumulation (140) de statistiques liées aux données d'usage, où les statistiques sont accumulées sur une période de temps et sont accessibles sur le compte de portail web ;
- l'évaluation (150) des statistiques en lien avec un critère de traitement ;
- l'association (160) des statistiques au compte de portail web pour un accès par un ou les deux parmi le patient et le professionnel de santé ; et
- la fourniture d'une recommandation de traitement au patient ou au professionnel de santé lorsqu'au moins l'une des statistiques correspond à ou dépasse un critère de traitement prédéterminé.

9. Système de prise en charge du diabète selon l'une quelconque des revendications 2 à 8 précédentes, dans lequel le ou les serveurs sont en outre configurés pour envoyer la recommandation de traitement au dispositif médical portable sous forme de message électronique.

10. Système de prise en charge du diabète selon la revendication 9, dans lequel le ou les serveurs sont en outre configurés pour solliciter une saisie provenant du professionnel de santé avant d'envoyer la recommandation de traitement à un dispositif informatique (20) du patient.

11. Système de prise en charge du diabète selon l'une quelconque des revendications 2 à 10 précédentes, dans lequel le ou les serveurs sont configurés de telle sorte que le professionnel de santé est en mesure de sélectionner le type de statistiques utilisées pour l'évaluation.

12. Procédé de partage d'informations et de prise en charge de données liées au diagnostic et de recommandations de traitement d'un patient souffrant d'une affection médicale, comprenant :
- la fourniture, au niveau d'un ou de plusieurs serveurs (18), d'un compte de portail web accessible à la fois par le patient et un professionnel de santé, où le compte de portail web est associé à un dispositif médical portable (28) ;
- la réception (130), au niveau d'un ou de plusieurs serveurs, de données médicales et de données d'usage provenant du dispositif médical portable, les données médicales étant associées au patient et les données d'usage indiquant l'usage du dispositif médical portable par le patient, dans lequel les données d'usage sont reçues par l'intermédiaire d'un composant de transfert de dispositif par le ou les serveurs et dans lequel les données d'usage incluent un code identifiant unique au dispositif médical portable (28) duquel lesdites données d'usage ont été reçues, dans lequel les données d'usage comprennent des données indiquant l'adhésion du patient au conseil de traitement par bolus, lesdites données comprenant un ou plusieurs parmi :
∘ le nombre et la chronologie des mesures de glycémie prises pendant une période spécifiée ;
∘ le nombre, la quantité et la chronologie des traitements par bolus administrés ;
∘ le nombre, la quantité et la chronologie des traitements correctifs administrés ;
- l'effacement de manière immédiate, par le composant de transfert de dispositif, d'une quelconque copie des données provenant du composant de transfert de dispositif après avoir envoyé avec succès lesdites données audit ou auxdits serveurs ;
- le recueil de données statistiques indiquant un nombre de fois que des données médicales ou des données d'usage stockées ailleurs dans un ou plusieurs dispositifs médicaux portables ont été synchronisées avec des données stockées dans le référentiel de données pour l'affichage dudit nombre de fois indiqué ;
- l'évaluation, par le ou les serveurs, si ledit code identifiant a déjà été stocké en association avec un compte dudit patient dans l'application de prise en charge du diabète (33), dans lequel ledit stockage indique un enregistrement réussi du dispositif médical portable identifié par ledit code identifiant pour ledit patient ;
- si ledit code identifiant a déjà été stocké en association avec ledit compte patient, le stockage de manière automatique, par le ou les serveurs, des données reçues dans le compte dudit patient dans le référentiel de données, utilisant de ce fait le code d'identification en tant que seule donnée d'identification nécessaire du patient et authentifiant de manière automatique le patient au niveau de l'application de prise en charge du diabète par l'intermédiaire dudit code d'identification ;
- si ledit code identifiant n'a pas déjà été stocké en association avec ledit compte patient, l'invite faite, par le ou les serveurs, au patient par l'intermédiaire d'une interface utilisateur de saisir les données d'authentification ; l'évaluation des données d'authentification saisies ; uniquement dans le cas d'une authentification réussie, le stockage automatique, par le ou les serveurs, des données reçues dans le compte dudit patient dans le référentiel de données, stockant de ce fait le code d'identification en association avec le compte du patient et enregistrant automatiquement ledit dispositif médical portable au niveau de l'application de prise en charge du diabète ;
- l'accumulation (140), au niveau du ou des serveurs, de statistiques liées aux données d'usage, où les statistiques sont accumulées sur une période de temps et sont accessibles sur le compte de portail web ;
- l'évaluation (150), au niveau d'un ou plusieurs serveurs, des statistiques en lien avec un critère de traitement ; et
- l'envoi (170), provenant d'un ou de plusieurs serveurs, d'une recommandation de traitement au patient ou au professionnel de santé lorsqu'au moins l'une des statistiques correspond au ou dépasse le critère de traitement.

13. Procédé selon la revendication 12 mis en oeuvre sur un système de prise en charge du diabète selon l'une quelconque des revendications 1 à 11.
